# EUROPEAN PATENT APPLICATION

(11) **EP 1 952 826 A1**
(43) Date of publication of application: **06.08.2008**
(21) Application number: 06822151.4
(22) Date of filing: 24.10.2006
(51) Int. Cl.: A61K 51/00, A61K 38/00, A61P 9/10, A61P 35/04, C07K 14/475, G01N 33/15, G01N 33/50, G01N 33/566

(54) **DIAGNOSTIC AGENT AND THERAPEUTIC AGENT FOR DISEASE ASSOCIATED WITH HEPATOCYTE GROWTH FACTOR RECEPTOR**

(30) Priority: 24.10.2005 JP 2005308852
(71) Applicant: Fujifilm RI Pharma Co., Ltd., Chuo-ku Tokyo 1040031 (JP)
(72) Inventor: NISHIMURA, Tsunehiko, Toyonaka-shi, Osaka 561-0861 (JP); NAGANO, Akio c/o Fujifilm RI Pharma Co., Ltd., Sanmu-shi, Chiba 289-1592 (JP)
(74) Representative: Blodig, Wolfgang
(86) International application number: PCT/JP2006/321171
(87) International publication number: WO 2007/049620

(57) **Abstract**

Disclosed is a diagnostic agent which is useful for imaging the site of application at which the highest therapeutic effect is expected (i.e., the site where an HGF receptor is expressed) in the therapy of an ischemic disease with an HGF plasmid for the purpose of promoting the angiogenesis with good efficiency.
The diagnostic agent is for a disease associated with a hepatocyte growth factor receptor, wherein the drug includes:
a labeled product of a partial polypeptide of hepatocyte growth factor containing a heparin-binding domain; or a labeled product of a polypeptide which consisting of an amino acid sequence in which one or several amino acid residues are substituted, deleted or added to the amino acid sequence of the partial polypeptide and having a heparin-binding activity

SEQUENCE LISTING

<110> Daiichi Radioisotope Laboratories, Ltd.

<120> Diagnostic or Therapeutic Agent for HGF-receptor-involved diseases

<130> P05741710

<160> 2

<170> PatentIn version 3.1

<210> 1
<211> 697
<212> PRT
<213> Homo sapiens
<221> DISULFID
<222> 39..65
<223> POTENTIAL
<221> DISULFID
<222> 43..53
<223> POTENTIAL
<221> DISULFID
<222> 97..175
<223> POTENTIAL
<221> DISULFID
<222> 118..158
<223> POTENTIAL
<221> DISULFID
<222> 146..170
<223> POTENTIAL
<221> DISULFID
<222> 456..573
<223> POTENTIAL
<221> CHAIN
<222> 1..463

<223>  Hepatocyte growth factor alpha chain.
<221>  CHAIN
<222>  464..697
<223>  Hepatocyte growth factor beta chain.

<400>  1

Gln Arg Lys Arg Arg Asn Thr Ile His Glu Phe Lys Lys Ser Ala Lys
1               5                   10                  15

Thr Thr Leu Ile Lys Ile Asp Pro Ala Leu Lys Ile Lys Thr Lys Lys
            20                  25                  30

Val Asn Thr Ala Asp Gln Cys Ala Asn Arg Cys Thr Arg Asn Lys Gly
        35                  40                  45

Leu Pro Phe Thr Cys Lys Ala Phe Val Phe Asp Lys Ala Arg Lys Gln
    50                  55                  60

Cys Leu Trp Phe Pro Phe Asn Ser Met Ser Ser Gly Val Lys Lys Glu
65                  70                  75                  80

Phe Gly His Glu Phe Asp Leu Tyr Glu Asn Lys Asp Tyr Ile Arg Asn
                85                  90                  95

Cys Ile Ile Gly Lys Gly Arg Ser Tyr Lys Gly Thr Val Ser Ile Thr
            100                 105                 110

Lys Ser Gly Ile Lys Cys Gln Pro Trp Ser Ser Met Ile Pro His Glu
        115                 120                 125

His Ser Phe Leu Pro Ser Ser Tyr Arg Gly Lys Asp Leu Gln Glu Asn
    130                 135                 140

Tyr Cys Arg Asn Pro Arg Gly Glu Glu Gly Gly Pro Trp Cys Phe Thr
145                 150                 155                 160

Ser Asn Pro Glu Val Arg Tyr Glu Val Cys Asp Ile Pro Gln Cys Ser
                165                 170                 175

Glu Val Glu Cys Met Thr Cys Asn Gly Glu Ser Tyr Arg Gly Leu Met
            180                 185                 190

Asp His Thr Glu Ser Gly Lys Ile Cys Gln Arg Trp Asp His Gln Thr
        195                 200                 205

Pro His Arg His Lys Phe Leu Pro Glu Arg Tyr Pro Asp Lys Gly Phe
    210                 215                 220

Asp Asp Asn Tyr Cys Arg Asn Pro Asp Gly Gln Pro Arg Pro Trp Cys
225                 230                 235                 240

Tyr Thr Leu Asp Pro His Thr Arg Trp Glu Tyr Cys Ala Ile Lys Thr
                245                 250                 255

Cys Ala Asp Asn Thr Met Asn Asp Thr Asp Val Pro Leu Glu Thr Thr
            260                 265                 270

Glu Cys Ile Gln Gly Gln Gly Glu Gly Tyr Arg Gly Thr Val Asn Thr
        275                 280                 285

Ile Trp Asn Gly Ile Pro Cys Gln Arg Trp Asp Ser Gln Tyr Pro His
    290                 295                 300

Glu His Asp Met Thr Pro Glu Asn Phe Lys Cys Lys Asp Leu Arg Glu
305                 310                 315                 320

Asn Tyr Cys Arg Asn Pro Asp Gly Ser Glu Ser Pro Trp Cys Phe Thr
                325                 330                 335

Thr Asp Pro Asn Ile Arg Val Gly Tyr Cys Ser Gln Ile Pro Asn Cys
            340                 345                 350

Asp Met Ser His Gly Gln Asp Cys Tyr Arg Gly Asn Gly Lys Asn Tyr
        355                 360                 365

Met Gly Asn Leu Ser Gln Thr Arg Ser Gly Leu Thr Cys Ser Met Trp
    370                 375                 380

Asp Lys Asn Met Glu Asp Leu His Arg His Ile Phe Trp Glu Pro Asp
385                 390                 395                 400

Ala Ser Lys Leu Asn Glu Asn Tyr Cys Arg Asn Pro Asp Asp Asp Ala
                405                 410                 415

His Gly Pro Trp Cys Tyr Thr Gly Asn Pro Leu Ile Pro Trp Asp Tyr
            420                 425                 430

Cys Pro Ile Ser Arg Cys Glu Gly Asp Thr Thr Pro Thr Ile Val Asn
        435                 440                 445

Leu Asp His Pro Val Ile Ser Cys Ala Lys Thr Lys Gln Leu Arg Val
    450                 455                 460

Val Asn Gly Ile Pro Thr Arg Thr Asn Ile Gly Trp Met Val Ser Leu
465                 470                 475                 480

Arg Tyr Arg Asn Lys His Ile Cys Gly Gly Ser Leu Ile Lys Glu Ser
                485                 490                 495

Trp Val Leu Thr Ala Arg Gln Cys Phe Pro Ser Arg Asp Leu Lys Asp
            500                 505                 510

Tyr Glu Ala Trp Leu Gly Ile His Asp Val His Gly Arg Gly Asp Glu
        515                 520                 525

Lys Cys Lys Gln Val Leu Asn Val Ser Gln Leu Val Tyr Gly Pro Glu
    530                 535                 540

Gly Ser Asp Leu Val Leu Met Lys Leu Ala Arg Pro Ala Val Leu Asp
545                 550                 555                 560

Asp Phe Val Ser Thr Ile Asp Leu Pro Asn Tyr Gly Cys Thr Ile Pro
                565                 570                 575

Glu Lys Thr Ser Cys Ser Val Tyr Gly Trp Gly Tyr Thr Gly Leu Ile
            580                 585                 590

Asn Tyr Asp Gly Leu Leu Arg Val Ala His Leu Tyr Ile Met Gly Asn
        595                 600                 605

Glu Lys Cys Ser Gln His His Arg Gly Lys Val Thr Leu Asn Glu Ser
    610                 615                 620

Glu Ile Cys Ala Gly Ala Glu Lys Ile Gly Ser Gly Pro Cys Glu Gly
625                 630                 635                 640

Asp Tyr Gly Gly Pro Leu Val Cys Glu Gln His Lys Met Arg Met Val
                645                 650                 655

Leu Gly Val Ile Val Pro Gly Arg Gly Cys Ala Ile Pro Asn Arg Pro
            660                 665                 670

Gly Ile Phe Val Arg Val Ala Tyr Tyr Ala Lys Trp Ile His Lys Ile
        675                 680                 685

Ile Leu Thr Tyr Lys Val Pro Gln Ser
    690                 695

<210> 2
<211> 73
<212> PRT
<213> Artificial

<220>
<223> Synthesized polypeptide
<221> DISULFID
<222> 39..65
<223> PROBABLE
<221> DISULFID
<222> 43..53
<223> PROBABLE
<400> 2

Phe Lys Lys Ser Ala Lys Thr Thr Leu Ile Lys Ile Asp Pro Ala Leu
1               5                   10                  15

Lys Ile Lys Thr Lys Lys Val Asn Thr Ala Asp Gln Cys Ala Asn Arg
            20                  25                  30

Cys Thr Arg Asn Lys Gly Leu Pro Phe Thr Cys Lys Ala Phe Val Phe
        35                  40                  45

Asp Lys Ala Arg Lys Gln Cys Leu Trp Phe Pro Phe Asn Ser Met Ser
    50                  55                  60

Ser Gly Val Lys Lys Glu Phe Gly His

65                  70

## Description

### Technical Field

The present invention relates to a reagent for detecting various diseases associated with a hepatocyte growth factor (HGF) receptor (c-Met) by imaging, and to a therapeutic drug for the diseases.

### Background Art

Hepatocyte growth factor (HGF) is a heterodimeric protein having a molecular weight of about 85 kDa in which an α-chain of about 69 kDa and a β-chain of 34 kDa are bound by a single disulfide bond. The α-chain is formed of a hairpin loop structure (amino acid sequence: Cys39 to Cys65) and four consecutive kringle structures (amino acid sequence: Cys97 to Cys438). The N-terminal region thereof containing the hairpin loop structure is known to bind to heparin (Non-Patent Document 1). The β-chain has a structure similar to that of serine protease (amino acid sequence: 495 to 728). The entire HGF has about 40% homology with plasminogen, but exhibits no protease activity. Human HGF is located on chromosome 7q21, and was cloned by Miyazawa, et *al.* (Non-Patent Documents 2 and 3).

HGF was originally considered as a growth factor regulating reconstitution of liver tissue after hepatectomy (Non-Patent Documents 4 and 5). However, HGF has been shown to exhibit a growth-promoting activity against, in addition to hepatocytes (liver tissue), various epithelial cells and endothelial cells, such as renal tubular epithelial cells (Non-Patent Document 6), mammary epithelial cells (Non-Patent Document 7), melanocytes (Non-Patent Document 8), epidermal cells (Non-Patent Document 9) and vascular endothelial cells (Non-Patent Documents 10 and 11), and some mesenchymal cells (Non-Patent Document 12).

HGF is known to exhibit a growth activity or a morphogenetic activity on epithelial cells expressing an HGF receptor after being produced by neighboring cells thereof such as hepatic nonparenchymal cells, hepatic or pulmonary endothelial cells, or a certain type of fibroblasts (Non-Patent Documents 13 to 19).

In the vascular system, HGF serves as a vascular-endothelium-specific growth factor which only stimulates the growth of vascular endothelial cells without affecting the growth of vascular smooth muscle cells (Non-Patent Document 20), and exhibits a neovascularization effect.

It has been known that HGF exhibits a growth effect, a cell motility-enhancing effect, a morphogenesis-inducing effect or a neovascularization effect in cancer cells, thereby inducing tumor invasion or metastasis in association with interstitial cells surrounding the cancer cells (Non-Patent Documents 21 and 22). Conversely, HGF may suppress growth of certain cancer cells to induce apoptosis (Non-Patent Document 23).

c-Met, which is an HGF receptor, was originally identified as an oncogene in human osteosarcoma cells transformed by a chemical carcinogen (Non-Patent Documents 24 and 25). c-Met is a heterodimeric protein of 190 kDa having transmembrane tyrosine kinase activity, in which an α-chain of 50 kDa and a β-chain of 145 kDa are bound by a disulfide bond (Non-Patent Document 26).

When c-Met binds to HGF, tyrosine kinase activity present in a cytoplasmic domain is activated. In response to tyrosine phosphorylation of c-Met, different cells exhibit different reactions including growth promotion, neovascularization, migration promotion, and morphogenesis. In normal tissue, such a biological activity promotes tissue repair. Meanwhile, in tumor tissue, invasion or metastasis of cancer cells is induced particularly by strongly promoting cell migration (Non-Patent Documents 31 and 32). Clinical studies have reported that the HGF receptor (c-Met) is highly expressed in cancer cells. Particularly, many c-Met studies have been focused and reported on gastric cancer (Non-Patent Documents 33 and 34), large intestinal cancer (Non-Patent Documents 35 and 36), oral squamous cell cancer (Non-Patent Document 37), thyroid cancer (Non-Patent Document 38) and so on. In such cancers, the positive rate of immunostaining for c-Met is considerably high (i.e., 60 to 100%). In addition, a relatively high positive rate has been reported for breast cancer, prostate cancer, non-small cell lung cancer, renal cell cancer, cervical cancer and others. It has also been reported that the expression level of c-Met is increased with cancer progression and that patients with increased expression level of c-Met result in poor prognosis. Therefore, the HGF receptor (c-Met) may be used as an index of malignancy.

The binding activity of HGF to c-Met has been reported to be derived from the N-terminal hairpin loop structure of the HGF molecule and a kringle structure following to the hairpin loop structure (Non-Patent Documents 27 to 30).

It has been observed that HGF binds not only to c-Met, but also to a certain type of heparin or heparan sulfate proteoglycan (HSPG) present on a cell surface or an extracellular matrix (Non-Patent Documents 39 and 40).

There have been reported several experiments for determining a heparin-binding site and a bioactive site in an HGF molecule. Mizuno, et al. have prepared a variety of deletion mutant HGFs: d-K1 (deletion of a first kringle domain); d-K2 (deletion of a second kringle domain); d-K3 (deletion of a third kringle domain); d-K4 (deletion of a fourth kringle domain); d-β (deletion of a β-chain); d-H (deletion of an N-terminal hairpin loop); and HK1K2 (a mutant consisting of the N-terminal hairpin loop and the first and second kringle domains), and have examined binding of these mutants to a heparin column (Non-Patent Document 43). As a result, they have reported that the mutants d-H and d-K2 exhibit reduced binding ability to a heparin affinity column, whereas native HGF and the other HGF mutants (d-K1, d-K3, d-K4, d-β, and HK1K2) bind to a heparin column. Aoyama, et *al.* have reported that a site including a truncated N-terminal hairpin loop structure (amino acid sequence: 70 to 96) binds to a heparin column (Non-Patent Document 1).
Matsumoto, *et al.* have reported that an N-terminal hairpin loop structure of HGF is essential for maintaining HGF bioactivities, since an HGF mutant in which the N-terminal hairpin loop structure has been deleted loses a hepatocyte-growth-promoting activity (i.e., an HGF bioactivity) (Non-Patent Document 27).

Current therapies for ischemic diseases (e.g., ischemic heart disease and peripheral vascular disease) include various drug therapies involving vasodilation; angioplasty using, for example, a catheter; and surgical bypass operation. The efficacy of these therapies has been sufficiently examined and therapeutic strategies using these therapies have been generally established.
However, there are problematic refractory ischemia including a case requiring reoperation even after these therapies, a case to which these therapies is not applicable, and a case which is not improved by these therapies.

Under such circumstances, regenerative medicine therapies have been focused in recent years, and basic and applied studies have been conducted therefor on a global scale. Regenerative medicine therapies include a revascularization therapy for regeneration of blood vessels in tissue and a tissue regeneration therapy. Among these therapies, a revascularization therapy has been focused to allow neovascularization for ensuring blood flow into ischemic tissue to reduce damage or necrosis of an ischemic region.
As revascularization therapies, clinically studied is a method employing a neovascularization-associated factor or a gene thereof, a method employing stem cells, or a cell mobilization therapy employing a cytokine.

In ischemic cardiomyocites or blood vessels of a subject with ischemic heart disease or in blood vessels of a subject with peripheral vascular disease, endogenous HGF level is lower than that in normal tissue and insufficient to rescue damaged blood vessels (Non-Patent Documents 45 and 46), and expression level of c-Met, an HGF-specific receptor present in vascular endothelial cells, is increased (Non-Patent Documents 47 to 53). Based on these findings, a gene therapy employing a plasmid or viral vector containing HGF protein or HGF gene has been developed to supply HGF to promote neovascularization.

On an animal model of ischemic heart disease or peripheral arterial disease, good therapeutic effects of the gene therapy employing a plasmid or viral vector containing HGF protein or HGF gene have been reported (Non-Patent Documents 54 to 63).
In clinical study, a gene therapy employing a plasmid containing HGF gene has been most often investigated. The safety and efficacy of the gene therapy were evaluated at Osaka University for patients with arteriosclerosis obliterans or Buerger's disease. As a result, it has been reported that the therapy generally caused no problem in safety and treated the diseases well (Non-Patent Document 44).
Currently, multi-center clinical trials (phase III) of the gene therapy are carried out in patients with peripheral vascular disease in Japan.

In the gene therapy employing a plasmid containing HGF gene, which targets patients with peripheral vascular disease, an ischemic region is determined through angiography by use of, for example, an X-ray or MRI contrasting agent, and a therapeutic drug is injected into the muscle around the ischemic region (Non-Patent Document 64).

In the case of ischemic heart disease, generally, an ischemic region is determined through, for example, angiography by use of an X-ray or MRI contrasting agent, a method employing a myocardial perfusion agent containing a radionuclide, or ultrasonography. Also, there is a less common method for determining the ischemic region by means of an NOGA system, in which the electric potential and spatial location of the endomyocardium are measured by means of a sensor provided at the tip of a catheter to determine the ischemic region on the basis of cardiac action potential and regional wall motion.

In a gene therapy employing an HGF plasmid, an ischemic region can be determined through any of the aforementioned conventional diagnostic imaging techniques such as angiography by use of an X-ray or MRI contrasting agent, a method employing a myocardial perfusion agent containing a radionuclide and an NOGA system, but the imaging techniques fail to directly image a c-Met expression site at which the highest therapeutic effect by administration is expected to be achieved.

Cancer diagnosis is performed by combination of an in vitro test and a diagnostic imaging technique. Examples of the diagnostic imaging technique include X-ray computed tomography (CT), magnetic resonance imaging (MRI), ultrasound imaging, and radionuclide scintigraphy. Administration of a contrasting agent to a patient provides a clear image through X-ray CT, MRI, or ultrasound imaging. Radionuclide scintigraphy requires administration of a radioactive drug product which is localized at a tumor site.

Such a cancer diagnostic imaging technique is generally based on determination of the presence of cancer, and currently the technique may detect a cancer of about several mm under specific conditions.
Meanwhile, there has not yet been developed a method for diagnosing cancer in view of its functions (characteristics), in particular, a method for determining the malignancy (e.g., invasion or metastasis) of cancer through imaging.

Many research groups have conducted studies on the relationship between overexpression of the HGF receptor (c-Met) and malignancy of human breast cancer or prostate cancer, and have reported the relationship between overexpression of the HGF receptor and the stage of breast cancer progression (Non-Patent Documents 76 to 78), and the relationship between overexpression of the HGF receptor and bone metastasis of prostate cancer (Non-Patent Documents 79 to 82).
The HGF receptor is a target molecule which can be used as an important indicator for evaluating the malignancy of cancer. Therefore, demand has arisen for development of a diagnostic drug which realizes determination of the presence and malignancy of a solid tumor by imaging expression of the HGF receptor in the solid tumor.

Cancer treatment is performed through, for example, a surgical therapy, a chemotherapy, a radiation therapy, or a multidisciplinary therapy incorporating these therapies.
There has been recently performed a tumor dormancy therapy in which cancer cells are maintained in a dormant state by use of a neovascularization inhibitor. In this therapy, tumor neovascularization is inhibited by a neovascularization inhibitor to block off a pathway for supplying nutrition and oxygen required for growth of cancer cells and induce cancer cell apoptosis, thereby maintaining cancer cells in a dormant state.
Bevacizumab (trade name: Avastin), which is a humanized monoclonal antibody preparation targeting a vascular endothelial growth factor (VEGF), has been approved as a neovascularization inhibitor in Europe and the United States and clinically used (the drug is under clinical trials in Japan). Examples of known neovascularization inhibitors under development include angiostatin (Non-Patent Document 65), endostatin (Non-Patent Document 66), and HGF antagonist NK4, which targets the HGF receptor (Patent Document 2 and Non-Patent Documents 67 to 70).
However, the tumor dormancy therapy can only induce dormancy of cancer cells, does not completely kill cancer cells. Thus the dormancy therapy is not for complete remission of cancer.
Similarly, NK4, a therapeutic drug targeting the HGF receptor, is not for complete remission of cancer. Therefore, there is a demand for the development of a therapeutic drug which targets a highly malignant tumor wherein the HGF receptor is expressed and allows for complete remission of the tumor.

A drug employing NK4 gene/protein has been studied, which has an N-terminal hairpin domain and four kringle domains of the α-chain of HGF (Patent Document 2). It has been reported that HGF labeled with a radionuclide (¹²⁵I) was used to examine in vivo kinetic parameter of HGF (Non-Patent Document 73) or affinity of HGF to heparan sulfate or dermatan sulfate (Non-Patent Document 74). It has also been reported is the tumor imaging by using an anti-c-Met monoclonal antibody labeled with a radionuclide (¹²⁵I) and c-Met-expressing cancer cells implanted into mice (Non-Patent Document 75).
Patent Document 1: WO 94/09969 pamphlet
Patent Document 2: JP-A-2003-250549
Non-Patent Document 1: Aoyama et al., Biochemistry 36: 10286-10291 (1997)
Non-Patent Document 2: Miyazawa et al., Biochem. Biophys. Res. Commun. 163(2): 967-973 (1989)
Non-Patent Document 3: Nakamura et al., Nature 342: 440-443 (1989)
Non-Patent Document 4: Nakamura et al., Biochem. Biophys. Res. Commun. 122(3): 1450-1459 (1984)
Non-Patent Document 5: Michalopoulos et al., Cancer Res. 44: 4414-4419 (1984)
Non-Patent Document 6: Igawa et al., Biochem. Biophys. Res. Commun. 174, 831-838 (1991)
Non-Patent Document 7: Niranjan et al., Development 121, 2897-2908 (1995)
Non-Patent Document 8: Rubin et al., Proc. Natl. Acad. Sci. USA 88, 415-419 (1991)
Non-Patent Document 9: Matsumoto et al., Exp. Cell Res. 196, 114-120 (1991)
Non-Patent Document 10: Bussolino et al., J. Cell Biol. 119, 629-641 (1992)
Non-Patent Document 11: Morimoto et al., Biochem. Biophys. Res. Commun. 179, 1042-1049 (1991)
Non-Patent Document 12: Gohda et al., Leuk. Lymphoma 19, 197-205 (1995)
Non-Patent Document 13: Noji et al., Biochem. Biophys. Res. Commun. 173, 42-47 (1990)
Non-Patent Document 14: Kinoshita et al., Biochem. Biophys. Res. Commun. 165, 1229-1234 (1989)
Non-Patent Document 15: Maher et al., J. Clin. Invest. 91, 2244-2252 (1993)
Non-Patent Document 16: Yanagita et al., Biochem. Biophys. Res. Commun. 182, 802-809 (1992)
Non-Patent Document 17: Rubin et al., Proc. Natl. Acad. Sci. USA 88, 415-419 (1991)
Non-Patent Document 18: Matsumoto et al., J. Biochem. 119: 591-600 (1996)
Non-Patent Document 19: Matsumoto et al., Biochem. Biophys. Res. Commun. 239: 639-644 (1997)
Non-Patent Document 20: Hayashi et al., Biochem. Biophys. Res. Commun 220: 539-545 (1996)
Non-Patent Document 21: Nakamura et al., Cancer Res. 57: 3305-3313 (1997)
Non-Patent Document 22: Jianq et al., Crit. Rev. Oncol. Hematol. 29: 209-248 (1999)
Non-Patent Document 23: Shima et al., Biochem. Biophys. Res. Commun. 180: 1151-1158 (1991)
Non-Patent Document 24: Bottaro et al., Science, 251: 802-804 (1991)
Non-Patent Document 25: Naldini et al., Oncogene, 6: 501-504 (1991)
Non-Patent Document 26: Park et al., Proc. Natl. Acad. Sci. USA, 84: 6379-6383 (1987)
Non-Patent Document 27: Matsumoto et al., Biochem. Biophys. Res. Commun., 181: 691-699 (1991)
Non-Patent Document 28: Hartmann et al., Proc. Natl. Acad. Sci., 89: 11574-11578 (1992)
Non-Patent Document 29: Lokker et al., EMBO J., 11: 2503-2510 (1992)
Non-Patent Document 30: Lokker et al., J. Biol. Chem., 268: 17145-17150 (1991)
Non-Patent Document 31: Jianq et al., Crit. Rev. Oncol. Hematol. 29: 209-248 (1999)
Non-Patent Document 32: Matsumoto et al., Kluwer. Acad. Pub. Chapter 6: 143-193 (2000)
Non-Patent Document 33: Tang et al., Oncol. Rep., 11: 333-339 (2004)
Non-Patent Document 34: Murakami et al., Int. Surg., 86: 151-157 (2001)
Non-Patent Document 35: Trovato et al., Eur. J. Histochem., 48: 291-297 (2004)
Non-Patent Document 36: Resnick et al., Clin. Cancer Res., 10: 3069-3075 (2004)
Non-Patent Document 37: Klosek et al., Oncol. Rep., 12: 293-296 (2004)
Non-Patent Document 38: Ippolito et al., Thyroid, 11: 783-787 (2001)
Non-Patent Document 39: Rouslahti et al., Cell 64: 867-869 (1991)
Non-Patent Document 40: Lyon et al., J. Biol. Chem. 269: 11216-11223 (1994)
Non-Patent Document 41: Zioncheck et al., J. Biol. Chem. 270: 16871-16878 (1995)
Non-Patent Document 42: Schwall et al., J. Cell Biol. 133: 709-718 (1996)
Non-Patent Document 43: Mizuno et al., J. Biol. Chem. 269: 1131-1136 (1994)
Non-Patent Document 44: Morishita et al., Hypertension 44: 203-209 (2004)
Non-Patent Document 45: Morishita et al., Hypertension 33: 1379-1384 (1999)
Non-Patent Document 46: Hayashi et al., Circulation 100: II301-8 (1999)
Non-Patent Document 47: Ueda et al., Ann. Thorac. Surg. 67: 1726-1731 (1999)
Non-Patent Document 48: Ueda et al., Cardiovasc. Res. 51: 41-50 (2001)
Non-Patent Document 49: Ono et al., Circulation 95: 2552-2558 (1997)
Non-Patent Document 50: Jin et al., J. Pharmacol. Exp. Ther. 304: 654-660 (2003)
Non-Patent Document 51: Liu et al., Microvasc. Res. 68: 156-160 (2004)
Non-Patent Document 52: Nakamura et al., J. Clin. Invest. 106: 1511-1519 (2000)
Non-Patent Document 53: Sato et al., Cardiovasc. Pathol. 10: 235-240 (2001)
Non-Patent Document 54: Aoki et al., Gene Therapy 7: 417-427 (2000)
Non-Patent Document 55: Kondo et al., J. Am. Coll. Cardiol. 44: 644-653 (2004)
Non-Patent Document 56: Li et al., Circulation 107: 2499-2506 (2003)
Non-Patent Document 57: Jayasankar et al., Circulation 108 [suppl. II]: II-230-II-236 (2003)
Non-Patent Document 58: Taniyama et al., Hypertension 40: 47-53 (2002)
Non-Patent Document 59: Hashiya et al., Igakuno Ayumi 210: 653-658(2004)
Non-Patent Document 60: Yoshiaki et al., Circulation 104: 2344-2350 (2001)
Non-Patent Document 61: Taniyama et al., Gene Ther. 8: 181-9 (2001)
Non-Patent Document 62: Belle et al., Circulation 97: 381-390 (1998)
Non-Patent Document 63: Morishita et al., Hypertension 33: 1379-1384 (1999)
Non-Patent Document 64: Morishita et al., Hypertension 44: 203-209 (2004)
Non-Patent Document 65: Cao et al., J. Clin. Invest. 101: 1055-1063 (1988)
Non-Patent Document 66: Blezinger et al., Nat. Biotechnol. 17: 343-348 (1999)
Non-Patent Document 67: Date et al., Oncogene 17: 3045-3054 (1998)
Non-Patent Document 68: Kuba et al., Cancer Res. 60: 6737-6743 (2000)
Non-Patent Document 69: Maehara et al., Br. J. Cancer 84: 864-873 (2001)
Non-Patent Document 70: Tomioka et al., Cancer Res. 61: 7518-7524 (2001)
Non-Patent Document 71: Baker et al., Life Sci. 49: 1583-91 (1991)
Non-Patent Document 72: Krenning et al., Eur. J. Nucl. Med.: 20, 716-31 (1993)
Non-Patent Document 73: Liu et al., Am. J. Physiol. 263: G642-9 (1992)
Non-Patent Document 74: Lyon et al., J. Biol. Chem. 269: 11216-11223 (1994) and J. Biol. Chem. 273: 271-278 (1998) Non-Patent Document 75: Hay et al., Clin. Cancer Res. Sep. 9: 3839S-3844S (2003)
Non-Patent Document 76: Niemann et al., J. Cell Biol., 143: 533-545 (1998)
Non-Patent Document 77: Tsarfaty et al., Anal. Quant. Cytol. Histol, 21: 397-408 (1999)
Non-Patent Document 78: Firon et al., Oncogene, 19: 2386-2397 (2000)
Non-Patent Document 79: Humphrey et al., Am. J. Pathol., 147: 386-396 (1995)
Non-Patent Document 80: Pisters et al., J. Urol., 154: 293-298 (1995)
Non-Patent Document 81: Watanabe et al., Cancer Lett., 141: 173-178 (1999)
Non-Patent Document 82: Knudsen et al., Urology, 60: 1113-1117 (2002)

### Disclosure of the Invention

### Problems to be Solved by the Invention

However, labeled HGF may promote effects of HGF; i.e., growth, invasion, and metastasis of cancer. In addition, labeled HGF poses problems in that, for example, a long period of time is required for imaging since labeled HGF has a high molecular weight and thus is slowly removed from blood, which causes long-term blood background in imaging. Further, a labeled anti-c-Met antibody, which has a molecular weight greater than that of labeled HGF, causes long-term blood background, and therefore poses problems in terms of, for example, antigenicity and requirement of a long period of time for imaging.

Therefore, an object of the present invention is to provide a diagnostic drug for an ischemic disease, wherein the drug realizes imaging of an administration site at which the highest therapeutic effect is expected to be achieved (i.e., an HGF receptor expression site) by efficiently promoting neovascularization in the treatment of the ischemic disease by use of a plasmid or virus vector containing HGF protein or HGF gene. Another object of the present invention is to provide a diagnostic or therapeutic drug for a cancer expressing the HGF receptor, wherein the drug allows to image a primary tumor or metastatic tumor of the cancer. Yet another object of the present invention is to provide a diagnostic or therapeutic drug effective for a disease involving neovascularization including cancer. Yet another object of the present invention is to provide a screening method for determining a therapeutic drug for a disease associated with the HGF receptor.

### Means for Solving the Problems

In order to achieve the aforementioned objects, the present inventors have conducted extensive studies, and as a result have found that a labeled product of a partial polypeptide of HGF containing a heparin-binding domain is accumulated specifically at the HGF receptor, and also at an HGF receptor expression site of a subject with ischemic disease or disease caused by neovascularization such as malignant tumor to image the site clearly, and that the labeled polypeptide is useful for the treatment of such a disease.

Accordingly, the present invention provides a diagnostic drug for a disease associated with a hepatocyte growth factor receptor, wherein the drug includes: a labeled product of a partial polypeptide of hepatocyte growth factor containing a heparin-binding domain; or a labeled product of a polypeptide which consisting of an amino acid sequence in which one or several amino acid residues are substituted, deleted or added to the amino acid sequence of the partial polypeptide, and having a heparin-binding activity.
The present invention also provides a therapeutic drug for a disease associated with a hepatocyte growth factor receptor, wherein the drug includes: a partial polypeptide of hepatocyte growth factor containing a heparin-binding domain; a polypeptide which consisting of an amino acid sequence in which one or several amino acid residues are substituted, deleted or added to the amino acid sequence of the partial polypeptide and having a heparin-binding activity; a labeled product thereof; or a toxin-bound product thereof.

The present invention also provides use of a labeled product of a partial polypeptide of hepatocyte growth factor containing a heparin-binding domain; or a labeled product of a polypeptide which consisting of an amino acid sequence in which one or several amino acid residues are substituted, deleted or added to the amino acid sequence of the partial polypeptide, and having a heparin-binding activity, for producing a diagnostic drug for a disease associated with a hepatocyte growth factor receptor.
The present invention also provides use of a partial polypeptide of hepatocyte growth factor containing a heparin-binding domain; a polypeptide which consisting of an amino acid sequence in which one or several amino acid residues are substituted, deleted or added to the amino acid sequence of the partial polypeptide and having a heparin-binding activity; a labeled product thereof; or a toxin-bound product thereof, for producing a therapeutic drug for a disease associated with a hepatocyte growth factor receptor.

The present invention also provides a method for diagnosing a disease associated with a hepatocyte growth factor receptor, wherein the method comprises administering a labeled product to a subject in need thereof; and detecting the labeled product, wherein the labeled product is a labeled product of a partial polypeptide of hepatocyte growth factor containing a heparin-binding domain or a labeled product of a polypeptide which consisting of an amino acid sequence in which one or several amino acid residues are substituted, deleted or added to the amino acid sequence of the partial polypeptide and having a heparin-binding activity.
The present invention also provides a method for treatment of a disease associated with a hepatocyte growth factor receptor, wherein the method comprises administering a partial polypeptide of hepatocyte growth factor containing a heparin-binding domain; a polypeptide which consisting of an amino acid sequence in which one or several amino acid residues are substituted, deleted or added to the amino acid sequence of the partial polypeptide and having a heparin-binding activity; a labeled product thereof; or a toxin-bound product thereof, to a subject in need thereof.
The present invention also provides a method for screening a preventive or therapeutic drug for a disease associated with a hepatocyte growth factor receptor, wherein the method comprises selecting a substance which inhibits binding of a hepatocyte growth factor receptor to a partial polypeptide of hepatocyte growth factor containing a heparin-binding domain; to a polypeptide which consisting of an amino acid sequence in which one or several amino acid residues are substituted, deleted or added to the amino acid sequence of the partial polypeptide and having a heparin-binding activity; or to a labeled product thereof. The present invention also provides a drug selected in the screening method.

### Effects of the Invention

Since a labeled polypeptide is accumulated specifically to the HGF receptor, only an HGF receptor expression site can be specifically and clearly imaged and a disease associated with the HGF receptor such as ischemic disease or a disease involving neovascularization (e.g., malignant tumor) can be imaged by using the diagnostic drug of the present invention. The diagnostic drug can also be employed for the treatment of such a disease.

### Brief Description of the Drawings

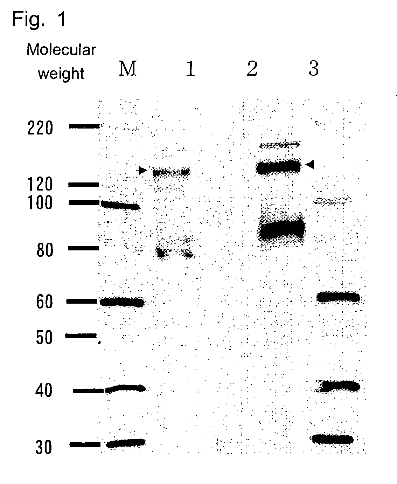

[Fig. 1] Fig. 1 shows the results of an experiment in which expression of c-Met in tumor cells was determined by use of DL-21 (mouse monoclonal anti-c-Met antibody).
   1: A431 Cell Lysate (commercially-available positive control)
   2: McA-RH 7777 homogenate
   3: HuCCT-1 homogenate
   M: Molecular weight marker

   c-Met (β-chain) was detected at a position of about 140 kDa (triangular mark) in a lane of HuCCT-1. c-Met was not detected in McA-RH 7777 because of specificity of the antibody (DL21 has specificity for human-derived c-Met).

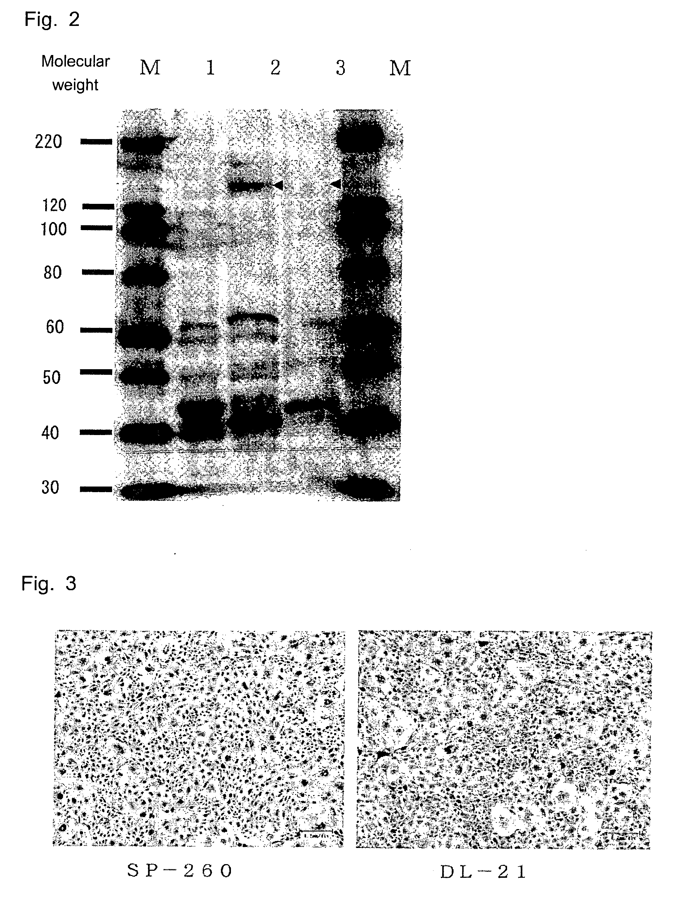

[Fig. 2] Fig. 2 shows the results of an experiment in which expression of c-Met in tumor cells was determined by use of SP260 (rabbit polyclonal anti-c-Met antibody).
   1: A431 Cell Lysate (commercially-available positive control)
   2: McA-RH 7777 homogenate
   3: HuCCT-1 homogenate
   M: Molecular weight marker

   c-Met was detected at a position of about 140 kDa (triangular mark) in a lane of SP260. Weak band of c-Met (β-chain) in HuCCT-1 lane is due to specificity of the antibody (SPF260 has high specificity for mouse- or rat-derived c-Met).
[Fig. 3] Fig. 3 shows images of c-Met-specific immunohistochemical staining using anti-c-Met antibodies (SP-260 and DL-21) and hematoxylin counterstaining.
[Fig. 4] Fig. 4 shows images of negative control for immunohistochemical staining and hematoxylin counterstaining.
[Fig. 5] Fig. 5 shows an image of accumulation of [¹²⁵I] HGF (11-83) NH₂ (McA-RH 7777).
[Fig. 6] Fig. 6 shows images of HE staining (McA-RH 7777) (right: enlarged image).
[Fig. 7] Fig. 7 shows images of immunohistochemical staining by use of an anti-c-Met antibody (SP-260) (McA-RH 7777) (right: enlarged image).
[Fig. 8] Fig. 8 shows the results of a binding inhibition experiment by use of hrHGF.
[Fig. 9] Fig. 9 shows a cardiac image obtained by use of ^{99m}Tc-MIBI.
[Fig. 10] Fig. 10 shows a cardiac image obtained by use of [¹²⁵I] HGF (11-83)-NH₂.
[Fig. 11] Fig. 11 shows an enlarged HE staining image corresponding to the boxed area of Fig. 9.
[Fig. 12] Fig. 12 shows an SPECT image of a myocardial ischemia rat model obtained by use of ^{99m}TC-MIBI.
[Fig. 13] Fig. 13 shows an SPECT image of a myocardial ischemia rat model obtained by use of [¹²³I] HGF (11-83)-NH₂.
[Fig. 14] Fig. 14 shows an SPECT image obtained by superimposing Figs. 12 and 13.

### Best Modes for Carrying Out the Invention

The diagnostic drug or therapeutic drug of the present invention is characterized by including a partial polypeptide of HGF containing a heparin-binding domain or a polypeptide which consisting of an amino acid sequence in which one or several amino acid residues are substituted, deleted or added to the amino acid sequence of the partial polypeptide and having a heparin-binding activity (hereinafter each of these polypeptides may be referred to as an "HGF-receptor-binding polypeptide"), or a labeled product thereof. HGF has several heparin-binding domains. Of these, a preferred domain is Cys39 to Cys65 of the amino acid sequence of HGF (SEQ ID NO: 1). The HGF-receptor-binding polypeptide employed in the present invention is preferably a polypeptide containing Cys39 to Cys65. Heparin-binding activity of such a polypeptide may be assayed by an experiment employing a heparin column as described in the Examples hereinbelow. The amino acid sequence of SEQ ID NO: 1 corresponds to the amino acid sequence of mature HGF which is formed by removing a signal peptide from an HGF precursor. Therefore, Gln1 of the amino acid sequence of SEQ ID NO: 1 corresponds to Gln32 of the amino acid sequence of the precursor containing the signal peptide.

The HGF-receptor-binding polypeptide encompasses, in addition to an HGF partial polypeptide, a polypeptide which consisting of an amino acid sequence in which one or several amino acid residues are substituted, deleted or added to the amino acid sequence of the partial polypeptide and having a heparin-binding activity (hereinafter the polypeptide having such an amino acid sequence may be referred to as a "modified product"). The above-described modification may be conducted by inducing mutation at one or several acid residues or by cleaving a coding sequence of the protein at predetermined amino acid residues using well known techniques in the art such as a technique based on polymerase chain reaction (PCR) or in vitro site-specific mutagenesis. See, for example, the method of Kunkel, *et al.* (Kunkel, et al., Pro. Natl. Acad. Sci. USA 82: 488-492 (1985)).

A modified HGF-receptor-binding polypeptide encompasses an HGF-receptor-binding polypeptide bound to another compound such as a polyethylene glycol (PEG) polymer. When a surface amino group of a polypeptide (e.g., an HGF-receptor-binding polypeptide) is modified with a PEG-containing compound, the circulation lifetime of the polypeptide is prolonged and the HGF-receptor-binding capability thereof can be maintained (Beauchamp, et al., Anal. Biochem. 131: 25-33 (1983)). No particular limitation is imposed on the molecular weight of PEG employed, but the molecular weight is generally 300 to 30,000, preferably 1,000 to 15,000. Modification of PEG may be performed through an arbitrary method well known in the art (e.g., Beauchamp, et al., Anal. Biochem. 131: 25-33 (1983)) .

The modified HGF-receptor-binding polypeptide within the scope of the present invention may be "treated" so as to contain an amino acid sequence which enables the polypeptide to be trapped on an affinity matrix. For example, a tag for supporting purification of the polypeptide such as c-myc, hemagglutinin, polyhistidine or Flag (registered trademark of Kodak) may be employed. The tag may be inserted into an arbitrary site in the polypeptide (including a carboxy terminus or an amino terminus). The HGF-receptor-binding polypeptide may be produced in a form fused with an enzyme facilitating detection of the polypeptide such as alkaline phosphatase.

Examples of preferred HGF-receptor-binding polypeptides include a polypeptide containing Cys39 to Cys65 and having four to 40 amino acid residues at the N-terminal side and/or four to 110 amino acid residues at the C-terminal side of the Cys39 to Cys65 region, and modified products of the polypeptide. Examples of more preferred HGF-receptor-binding polypeptides include a polypeptide containing Cys39 to Cys65 and having 4 to 40 amino acid residues at the N-terminal side and/or the C-terminal side of the Cys39 to Cys65 region, and modified products of the polypeptide. Examples of most preferred HGF-receptor-binding polypeptides include a polypeptide containing Cys39 to Cys65, and having 4 to 30 amino acid residues at the N-terminal side and/or the C-terminal side of the Cys39 to Cys65 region; and modified products of the polypeptide. Specific examples of such preferred polypeptides include polypeptides include Cys39 to His83, His9 to Glu80, Phe11 to His83 (SEQ ID NO: 2), Gln1 to His83, Phe11 to Asn96, Gln1 to Asn96 and Gln1 to Cys175, of the amino acid sequence of SEQ ID NO: 1.
Of these, a polypeptide having the amino acid sequence of SEQ ID NO: 2, or a modified product thereof is particularly preferred.

The modified HGF-receptor-binding polypeptide employed in the present invention includes a polypeptide in which an amino acid residue(s) is substituted, deleted or added and having a heparin-binding activity as described above. Generally, such a modified product preferably has a homology of 90% or more, more preferably 95% or more with the HGF partial polypeptide. Meanwhile, the heparin-binding activity of the modified product is preferably 10% or more, more preferably 50% or more, still more preferably 60% or more, yet still more preferably 70% or more, particularly preferably 80% or more, of the heparin-binding activity of the polypeptide Cys39 to Cys65.

The labeled product of an HGF-receptor-binding polypeptide includes any detectable forms which are generally used for diagnosis. That is, for diagnostic use, the HGF-receptor-binding polypeptide may be labeled with a detectable moiety. Such a detectable moiety can produce a detectable signal directly or indirectly. Examples of the detectable moiety may include radionuclides such as ³H, ¹⁴C, ³²P, ³⁵S and ¹²⁵I; fluorescent or chemiluminescent compounds such as fluorescein isothiocyanate, rhodamine and luciferin; and enzymes such as alkaline phosphatase, β-galactosidase and horseradish peroxidase. Binding of the polypeptide to the detectable moiety may be performed with a method well known in the art. See, for example, Hunter, et al., Nature 144: 945 (1962); David, et al., Biochemistry 13: 1014 (1974); Pain, et al., J. Immunol. Meth. 40: 219 (1982); and Nygren, Histochem. and Cytochem. 30: 407 (1982).

For imaging of the HGF receptor, the HGF-receptor-binding polypeptide may be bound to a detectable moiety useful for in vivo imaging.
The HGF-receptor-binding peptide is labeled with a detectable moiety such as a radionuclide, a radiopaque agent, a paramagnetic agent or a surfactant. The labeled HGF-receptor-binding polypeptide is administered to a mammal, preferably into blood flow, and the presence and location of the labeled polypeptide are detected from outside. The HGF-receptor-binding polypeptide may also be labeled with any moieties for the polypeptide which can be detected in a mammal with other methods such as nuclear magnetic resonance, radiochemistry, ultrasonograph or any other methods well known in the art. An exemplified labeled polypeptide consists of an HGF-receptor-binding portion of the polypeptide, an optional linking group, and a detectable moiety. The detectable moiety includes a γ-ray- or positron-emitting radioactive diagnostic agent, a contrasting agent for nuclear magnetic resonance diagnostic imaging, an X-ray contrast agent and an ultrasonic contrast agent.

More specifically, the exemplified labeled polypeptide include a radioactive diagnostic agent in which a polypeptide structure or an amino acid residue (e.g., tyrosine residue or histidine residue) of the polypeptide is directly labeled with one or more radionuclides such as ¹¹C, ¹³N, ¹⁵O, ¹⁸F, ^{34m}Cl, ³⁸Cl, ⁷⁵Br, ⁷⁶Br, ⁷⁷Br, ^{80m}Br, ⁸⁰Br, ⁸²Br, ¹²¹I, ¹²³I, ¹²⁴I, ¹²⁶, and ¹³¹I); an X-ray diagnostic agent containing one or more atoms which absorb X-ray (i.e., an atom(s) having an atomic number of 20 or more); and a contrasting agent for nuclear magnetic resonance diagnosis including a paramagnetic compound such as nitroxide).

Alternatively, the exemplified labeled polypeptide includes a contrasting agent in the form of a metal complex of the polypeptide obtained by introducing a linking group such as a bifunctional ligand or a carbonyl compound into the polypeptide. The bifunctional ligand is preferably a polyaminopolycarboxylic acid. The polyaminocarboxylic acid is selected from the group consisting of ethylenediaminetetraacetic acid (EDTA), diethylenetriaminepentaacetic acid (DTPA), diethylenetriaminepentaacetic acid-bismethylamide (DTPA-BMA), 1,4,7,10-tetraazacyclododecane-1,4,7,10-tetraacetic acid (DOTA), and derivatives thereof. The bifunctional ligand other than the polyaminopolycarboxylic acid is selected from, for example, the group consisting of 6-hydrazinonicotinamide (HYNIC) and derivatives thereof.

The exemplified labeled polypeptide also includes a complex useful as a nuclear magnetic resonance diagnostic agent, such as a metal complex of the polypeptide and the bifunctional-ligand which having, as a metal component, a metal ion exhibiting paramagnetic property due to unpaired electrons in inner shell thereof (e.g., a paramagnetic ion of metal element selected from the group consisting of Co, Mn, Cu, Cr, Ni, V, Au, Fe, Eu, Gd, Dy, Tb, Ho and Er).
The exemplified labeled polypeptide also includes a complex useful as an X-ray diagnostic agent, such as a metal complex of the polypeptide and the bifunctional-ligand which having, as a metal component, a metal ion which absorbs X-ray, specifically a metal ion of a metal element having an atomic number of 20 or more (e.g., a metal element selected from the group consisting of Re, Sm, Ho, Lu, Pm, Y, Bi, Pb, Os, Pd, Gd, La, Au, Yb, Dy, Cu, Rh, Ag and Ir).
The exemplified labeled polypeptide also includes a complex useful as a radioactive diagnostic agent, such as a metal complex of the polypeptide and the bifunctional-ligand having, as a metal component, an ion of a radionuclide selected from the group consisting of ⁴⁷Sc, ^{52m}Mn, ⁵⁵Co, ⁶²Cu, ⁶⁴Cu, ⁶⁷Cu, ⁶⁷Ga, ⁶⁸Ga, ⁷²As, ⁷²Se, ⁷³Se, ⁷⁵Se, ⁷⁶As, ⁹⁵Tc, ^{99m}Tc, ¹⁰⁵Rh, ¹⁰⁹Pd, ¹¹¹In, ¹⁵³Sm, ¹⁷⁷Lu, ¹⁸⁶Re, ¹⁸⁸Re, ¹⁹⁸Au, ¹⁹⁹Au, ²⁰¹Tl, ²¹¹At and ²¹²Bi.

According to the present invention, the polypeptide is preferably labeled at a site other than Cys39 to Cys65.

The HGF-receptor-binding polypeptide bound to a radionuclide emitting β-rays, α-rays, or Auger or Coster-Kronig electrons may be employed as a therapeutic drug targeting the HGF receptor.
Examples of radionuclides suitable for therapeutic application include ⁴⁷Sc, ⁶⁷Cu, ⁸⁹Sr, ⁹⁰Y, ¹⁰³Pd, ¹⁰⁵Rh, ¹⁰⁹Pd, ¹¹¹Ag, ¹²⁵I, ¹³¹I, ¹⁴⁰La, ¹⁴⁹Pm, ¹⁵³Sm, ¹⁵⁹Gd, ¹⁶⁵Dy, ¹⁶⁶Dy, ¹⁶⁶Ho, ¹⁶⁹Yb, ¹⁷⁵Yb, ¹⁷⁷Lu, ¹⁸⁶Re, ¹⁸⁸Re, ¹⁹²Ir, ¹⁹⁸Au, ¹⁹⁹Au, ²¹¹At, ²¹²Bi, ²¹²Pb, and ²¹⁷Bi. Such a radionuclide is selected so as to form a stable complex with the polypeptide.

Alternatively, the exemplified labeled polypeptide includes an ultrasonic contrast agent which containing a surfactant moiety bound to a polypeptide moiety and including ultrafine biocompatible gas bubbles, a liquid carrier or surfactant microspheres.

The HGF-receptor-binding polypeptide bound to a toxin may be employed as a therapeutic drug targeting the HGF receptor. Using a procedure well known in the art, the HGF-receptor-binding polypeptide may be bound to a toxic polypeptide which mediates cytotoxic effects in cell cytoplasm. Examples of preferred toxic polypeptides include ribosome-inactivating proteins; plant-derived toxins such as A-chain toxin (e.g., ricin A-chain), saporin, bryodin, gelonin, abrin and pokeweed antiviral protein (PAP); fungal toxins such as α-sarcin, aspergillin and restrictocin; bacterial toxins such as diphtheria toxin (DT); pseudomonas exotoxin A; and ribonucleic acids such as placental ribonucleic acid and angiogenin. Examples of other useful toxic polypeptides include apoptosis-promoting polypeptides such as Fas ligand, TRAIL, TNF-α, TNF-β, Apo-3 ligand, Bax, Bad, Bak, Bim, Bik, Bok and Hrk. A plurality of (e.g., 2, 3, 4, 6, 8, 10, 15, or 20) functional fragments of one or more (e.g., 2, 3, 4, or 6) toxins may be bound to the HGF-receptor-binding polypeptide. When functional fragments have repetitive sequence, such repetitive regions may locate adjacently each other, or may be separated from each other by one or more target fragments or by any of the aforementioned binding peptides.

An HGF-receptor-binding polypeptide employed in the present invention may be prepared by, for example, degrading HGF proteins with an enzyme or others, or using tissue culture cells expressing a target polypeptide of interest. A substantially pure polypeptide may be obtained through a polypeptide purification technique such as affinity chromatography or HPLC. Alternatively, the HGF-receptor-binding polypeptide may be produced with a polypeptide synthesis technique. Examples of polypeptide synthesis techniques include solid phase synthesis and liquid-phase synthesis *("*Jikken Kagaku Koza (Experimental Chemical Course), 5th edition, Vol. 16, Yuki Kagobutsu no Gosei (Synthesis of Organic Compound) IV Carboxylic Acid, Amino Acid, Peptide," edited by The Chemical Society of Japan, Tokyo Kagaku Dojin, Co., Ltd. (2005)).

The HGF-receptor-binding polypeptide may be produced using a recombination technique. The cDNA sequence of human HGF is known (Miyazawa, et al., Biochem. Biophys. Res. Commun. 163: 967-973 (1989); Nakamura, et al., Nature 342: 440-443 (1989)). Methods for inducing protein expression from cDNA to obtain a protein product are well known. See, for example, Maniatis, et al., 1989, "Molecular Cloning: A Laboratory Manual," Cold Spring Harbor Laboratory, New York, pp. 16.1 to 17.44. Examples of suitable expression systems include, but are not limited to, microorganisms such as bacteria (e.g., E. coli and *B. subtilis*) transformed with expression vectors from recombinant bacteriophage, plasmid DNA or cosmid DNA; yeast (e.g., *Saccharomyces* and *Pichia)* transformed with recombinant yeast expression vectors; insect cell systems infected with recombinant virus expression vectors (e.g., baculovirus); plant cell systems infected with recombinant virus expression vectors (e.g., cauliflower mosaic virus (CaMV) and tobacco mosaic virus (TMV)) containing fusion protein nucleotide sequences, or transformed with recombinant plasmid expression vectors (e.g., Ti plasmid) containing fusion protein nucleotide sequences; and mammalian cell systems (e.g., HEK, COS, CHO, BHK, 293, VERO, HeLa, MDCK, WI38, and NIH 3T3 cells) transformed with expression constructs containing promoters derived from the genome of mammalian cells (e.g., metallothionein promoter) or promoters derived from mammalian viruses (e.g., adenovirus late promoter and vaccinia virus 7.5K promoter). Examples of useful host cells include primary or secondary cells obtained directly from a mammal, transformed with a plasmid vector, or infected with a viral vector (e.g., in particular, herpes virus, herpes simplex virus, retrovirus, vaccinia virus, attenuated vaccinia virus, canarypox virus, adenovirus, adeno-associated virus, lentivirus, poxvirus, lentivirus (HIV), Sendai virus, Epstein-Barr virus (EBV), poliovirus, Sindbis virus, and simian virus 40 (SV40)).

Target disease of the diagnostic drug or therapeutic drug of the present invention includes HGF-receptor-associated diseases, such as ischemic diseases and malignant tumors. Examples of ischemic diseases include ischemic heart diseases and peripheral vascular diseases as described above. Examples of malignant tumors include ovarian cancer, pancreatic cancer, gastric cancer, gallbladder cancer, kidney cancer, prostate cancer, breast cancer, esophageal cancer, liver cancer, oral cancer, colon cancer, large intestinal cancer, sarcoma, glioma and melanoma. The diagnostic drug or therapeutic drug of the present invention is also applicable for diagnosis or treatment of pathological conditions associated with HGF receptor expression and angiogenesis, such as myocardial angiogenesis, diabetic retinopathy, diabetic neovascularization, inadequate wound healing, and inflammatory disease.

The diagnostic drug or therapeutic drug of the present invention is most preferably administered via intravenous injection. However, the drug may be administered through other common routes such as oral, intraarterial, intramuscular, subcutaneous, intradermal, intraarticular, and intrasynovial.
The dose of the diagnostic drug or therapeutic drug of the present invention is, for example as a radioactive drug, appropriately determined depending on various conditions such as the body weight, age and sex of a patient, or type of the measuring apparatus employed (e.g., an SPECT apparatus). In general, the dose of the diagnostic drug is 111 MBq to 222 MBq as reduced to radioactivity of ¹²³I, whereas the dose of the therapeutic drug is 37 MBq to 3,700 MBq as reduced to radioactivity of ¹³¹I.
In an imaging method employing the diagnostic drug of the present invention, when, for example, ¹²³I is employed, preferably, the drug is intravenously administered to a subject at a dose within the aforementioned range, and about one hour thereafter, the whole body or the surrounding area of the lesion of the subject is subjected to time-course planar imaging or tomography (SPECT) by means of a γ-camera.

A preparation of the diagnostic drug or therapeutic drug of the present invention may prepared by adding a stabilizer such as propylene glycol, a pH-adjusting agent such as an acid or a base, a buffer such as a phosphate buffer or an isotonizing agent such as saline, to the aforementioned active ingredient.

The aforementioned HGF-receptor-binding polypeptide or the labeled product thereof can be employed for screening of a preventive or therapeutic drug for HGF-receptor-associated diseases. Specifically, the HGF-receptor-binding polypeptide or the labeled product thereof is employed for selecting a substance which inhibits binding between the polypeptide or the labeled product thereof and the HGF receptor. Such a binding-inhibitory substance is useful as a preventive or therapeutic drug for HGF-receptor-associated diseases. The binding-inhibitory substance may be selected with a generally employed binding inhibition test. For example, the labeled HGF-receptor-binding polypeptide is reacted with the HGF receptor in the presence of a test substance, and the amount of the labeled HGF-receptor-binding polypeptide bound (or not bound) to the HGF receptor is measured.
The thus-selected binding-inhibitory substance is useful as a preventive or therapeutic drug for HGF-receptor-associated diseases.

### Examples

The present invention will be described hereinafter in more detail by way of examples, which should not be construed as limiting the invention thereto.

### Example 1

By means of an automated peptide synthesizer based on the solid phase method, a polypeptide represented by SEQ ID NO: 2 (HGF(11-83)-NH₂) was synthesized.

### <High-performance liquid chromatography conditions>

Column: WMC-Pack R-ODS-5-A (250 x 4.6 mm)
Mobile phase:
   solution A: 0.1% TFA in water;
   solution B: 0.1% TFA in water/AcCN (30/70);
   25 min linear gradient 0% to 100% (solution B)
Flow rate: 1.0 mL/min
Temperature: 35°C
Detector: UV: 210 nm 20. AUFS
Purity: ≥95%

### <Mass analysis>

Measured (MALDI-TOF): 8295.71 (calcd.: 8294.86[M+H]⁺)

### Example 2

### Synthesis of [¹²⁵I]HGF(11-83)-NH₂ (73 aa)

The labeled product was prepared through directly introducing [¹²⁵I] into HGF(11-83)-NH₂ (73 aa) (Lot: HGF11-83-NH₂) produced in Example 1.

To a solution mixture containing a 1.0 mg/mL aqueous solution (25 µL) of HGF(11-83)-NH₂ (73 aa) trifluoroacetic acid salt, a 0.5M sodium phosphate buffer (pH 7.0) (50 µL), and a [¹²⁵I] sodium iodide solution (1 to 10 mCi) (10 to 20 µL), a 0.25 mg/mL aqueous sodium p-toluenesulfonchloramide solution (10 µL) was added, and the mixture was allowed to stand at room temperature for one hour. This reaction was repeated several times. All the yielded reaction mixtures were chromatographically purified using a reverse phase column (YMC-Pack R-ODS-5-A, 4.6 × 250 mm) at a flow rate of 1.0 mL/min with a mobile phase containing Solution A (0.1% aqueous trifluoroacetic acid) and solution B (0.1% aqueous trifluoroacetic acid/acetonitrile 30/70 mixture) (0 to 50 min linear gradient, solution A: 100% to 0%). During purification, 10% aqueous bovine serum albumin (100 µL) was added as a stabilizer. The organic solvent was removed from the recovered fraction at room temperature under reduced pressure, and 10% aqueous bovine serum albumin and water were added in appropriate amounts so as to obtain 1% aqueous bovine serum albumin of about 0.5 mCi/mL, followed by filtration by means of a 0.20 µm membrane filter, to thereby prepare a solution of interest. This solution was stored at -20°C for up to eight weeks in order to be employed in the subsequent experiments; i.e., heparin-binding activity measurement, determination of distribution in a tumor-cell-transplanted model animal, and determination of distribution in an ischemic heart disease model animal. When chromatographic analysis under the conditions employing the reverse phase column was performed, the radiochemical purity of free [¹²⁵I], which is a decomposed product, was 10% or less.

### Example 3

### Observation of heparin-binding activity of [¹²⁵I]HGF(11-83)-NH₂ (73 aa) by means of a heparin affinity column

The heparin-binding activity of [¹²⁵I]HGF(11-83)-NH₂ (73 aa) produced in Example 1 was determined with high-performance liquid chromatography employing a heparin column.
hrHGF (PEPTIDE INSTITUTE, INC.), having a high heparin-binding activity, was used as a positive control.

### <High-performance liquid chromatography conditions>

Column: TSK-gel Heparin-5PW (0.75 x 7.5 cm): product of TOHSO
Mobile phase:
   solution A: 10mM Na phosphate buffer (pH 7.5);
   solution B: 10mM Na phosphate buffer (pH 7.5) + 2M NaCl;
   0 min to 3 min: solution A 100%
   3 min to 30 min: linear gradient solution B 0% to 100%
Flow rate: 0.7 mL/min
Temperature: room temperature (about 25°C)
Detector: 215 nm and 280 nm UV, RI
hrHGF was eluted at a retention time of 23.4 min with NaCl (about 1.5M).

[¹²⁵I]HGF(11-83)-NH₂ (73 aa) was eluted at a retention time of 20.3 min with NaCl of about 1.3M, showing high heparin-binding activity. In addition to [¹²⁵I]HGF(11-83)-NH₂ (73 aa), an elution peak attributed to free ¹²⁵I and a peak which might attribute to a peptide with reduced heparin-binding activity due to decomposition or denature were observed at 4.3 min and 9.9 min, respectively. Area ratios of the three peaks were 78.1%, 3.1%, and 18.8%, indicating that about 80% of the sample had a strong heparin-binding activity.

### Example 4

### Accumulation of [¹²⁵I]HGF(11-83)-NH₂ (73 aa) in a c-Met expressing tumor

c-Met-specific accumulation of [¹²⁵I]HGF(11-83)-NH₂ (73 aa) in a tumor was observed by autoradiography and immunohistostaining in mice subcutaneously implanted c-Met expressing cancer cells.

### 1. Determination of c-Met expression in tumor cells employed in the experiment

Expression of c-Met in McA-RH7777 cells (rat-origin liver cancer cells) and in HuCCT-1 cells (human-origin cholangiocarcinoma cells) were observed by Western blotting. Specifically, HuCCT-1 cells and McA-RH7777 cells were cultured, and a homogenate (protein content: 1.0 mg/mL) was prepared from each of the cultures. Each sample was purified by SDS-PAGE (7.5% gel) and transferred to a PVDF membrane. The transferred membrane was reacted with a primary antibody DL21 (mouse monoclonal anti-c-Met antibody; Upstate) or SP260 (rabbit polyclonal anti-c-Met antibody; Santa Cruz Biochemistry) and with a secondary antibody (ECL Anti-mouse IgG; Amersham Biosciences, or ECL Anti-rabbit IgG; Amersham Biosciences), followed by detection by means of an ECL detection Kit (Amersham Biosciences). When the DL21 antibody was employed, a band was detected in HuCCT1 lane at a position of the expected molecular weight, i.e., about 140 kDa (Fig. 1), whereas when the SP260 antibody was employed, a 140 kDa band was detected in McA-RH7777 lane and a weak band was detected in HuCCT1 lane (Fig. 2). Thus, c-Met was found to be expressed in both McA-RH7777 cells and HuCCT-1 cells.

### 2. Identification of c-Met-expressed cancer cells by immunohistostaining

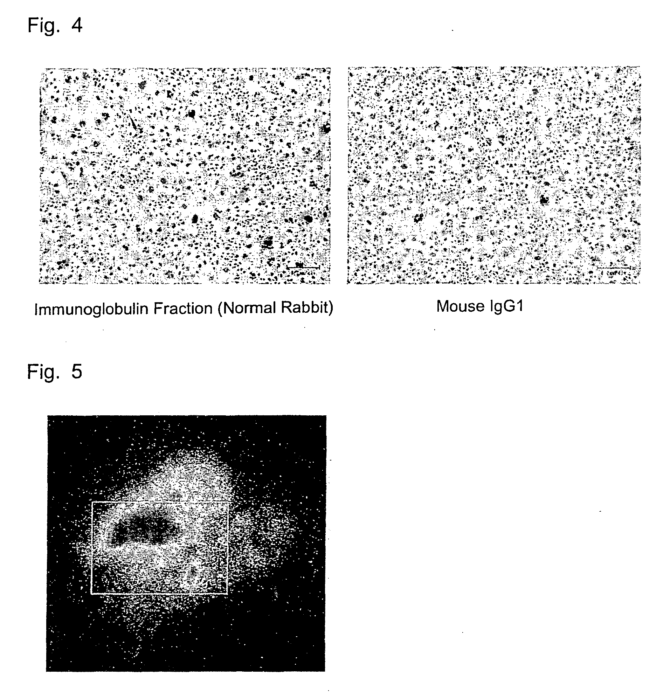

c-Met-expressed HuCCT1 cells (human-origin cholangiocarcinoma cells) were cultured by means of a LabTek slide chamber (NUNC). The cultured cells were fixed with 10% formalin buffer and preliminarily treated with 0.2% Triton X-100. The cells were reacted with an anti-c-Met antibody (SP-260 or DL-21) as a primary antibody, and with an unimmunized normal rabbit immunoglobulin fraction (DAKO) as a negative control of SP-260 or a mouse IgG1 (DAKO) as a negative control of DL-21 at room temperature for two hours. Subsequently, Simple stain rat MAX-PO(R) (NICHIREI) was added to a sample treated with SP-260 and an unimmunized normal rabbit immunoglobulin fraction, and Simple stain rat MAX-PO(M) (NICHIREI) was added to a sample treated with DL-21 and a mouse IgG1. After reacting at room temperature for 30 minutes, a DAB substrate solution (DAKO) was added to each sample and the each mixture was reacted at room temperature for 10 minutes for immunohistostaining. Subsequently, counterstaining with hematoxylin was performed (Figs. 3 and 4) .
As a result, c-Met-specific staining by anti-c-Met antibodies was observed.

### 3. Observation of c-Met-specific accumulation of [¹²⁵I]HGF(11-83) -NH₂ (73 aa) in a tumor of mice subcutaneously implanted c-Met expressing cancer cells by autoradiography and immunohistostaining

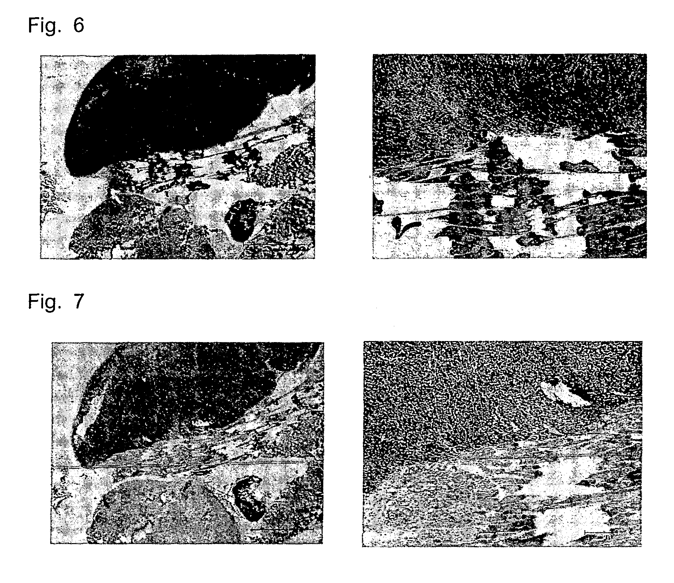

A suspension of c-Met-expressed McA-RH7777 cells (rat-origin liver cancer cells) (200 µL, 2.0 × 10⁶ cells) was subcutaneously injected to a right hindlimb of 6-week-old nude mice (BALB/c nu/nu: Japan SLC, Inc.). On day 9 (tumor volume: 82 to 478 cm³ (McA-RH7777 cells)), the aforementioned labeled HGF-fragment (370 kBq/100 µL) was intravenously administered to each mouse. The tumor was isolated with muscles surrounding the tumor 15 minutes after administration, and frozen sections were prepared. Each frozen section was affixed to an imaging plate (Fuji Photo Film Co., Ltd) for seven days, and a [¹²⁵I]HGF(11-83)-NH₂ (73 aa) accumulation image was observed by means of a bio-imaging analyzer BAS-1800 (Fuji Photo Film Co., Ltd) (Fig. 5). One analyzed image revealed that [¹²⁵I]HGF(11-83)-NH₂ (73 aa) was accumulated in the tumor region at higher level compared to the surrounding muscles. The accumulation level in the tumor was found to be 4.6 to 8.1 times greater than that in the surrounding muscles. Serial frozen sections were subjected to hematoxylin-eosin (HE) staining (Fig. 6) and immunohistochemical staining with anti-c-Met antibody (SP-260) (Fig. 7). As a result, c-Met-specific staining was observed in the tumor region, which corresponded to the result from the [¹²⁵I]HGF(11-83)-NH₂ (73 aa) accumulation image.
Therefore, accumulation of [¹²⁵I]HGF(11-83)-NH₂ (73 aa) was indicated to be c-Met-specific.

### Example 5

### Determination of binding of [¹²⁵I]HGF(11-83)-NH₂(73 aa) to HGF receptor

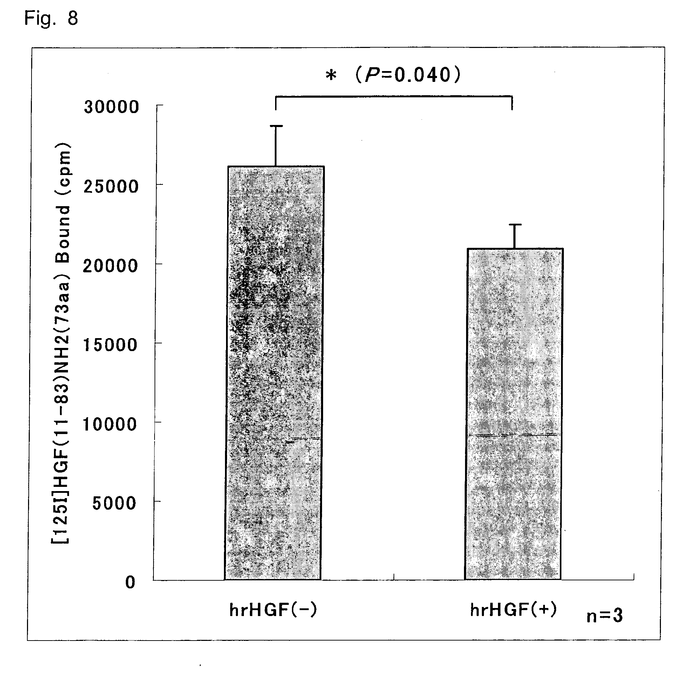

Inhibition of binding of [¹²⁵I]HGF(11-83)-NH₂ (73 aa) to c-Met-expressing HuCCT-1 cells (human-origin cholangiocarcinoma cells) by human recombinant HGF (hrHGF) was determined by the binding assay.
Specifically, a suspension of HuCCT-1 cells (100 µL, (1.5 × 10⁵ cells)) prepared for a binding inhibition test was placed in an Eppendorf tube whose inner surface had been coated with bovine serum albumin (BSA), and a solution of hrHGF (PEPTIDE INSTITUTE, INC.) (50 µL, final concentration: 379 nM) was added to the tube. A negative control was prepared by adding a binding buffer (0.2% BSA, 20mM HEPES Hanks' solution, pH 7.0) (50 µL) instead of the rhHGF solution. To the samples, 0.758 nM [¹²⁵I]HGF(11-83)-NH₂ (73 aa) solution (50 µL) (final concentration: 0.190 nM, ratio by amount to hrHGF: 1:2,000) was added. Each Eppendorf tube containing HuCCT-1 cells was stirred and incubated at room temperature for one hour, followed by centrifuged at 4°C (3,000 rpm, 3 min) to precipitate the cells. The supernatant was removed by means of an aspirator. The cells were stirred with an ice-cooled binding buffer (500 µL), and the mixture was centrifuged (3,000 rpm, 3 min). The procedure was repeated thrice. The radioactivity of the Eppendorf tube containing HuCCT-1 cells was measured by means of a gamma counter. The average and the standard deviation of the radioactivity (cpm) were calculated for the samples to which the hrHGF solution had been added (hrHGF(+)) and for the samples to which the binding buffer had been added (hrHGF(-)). The difference between the averages was assessed through the Student's t-test.
As a result, the radioactivity attributed to [¹²⁵I]HGF(11-83)-NH₂ (73 aa) bound to HuCCT1 cells was 26136.2 ± 2580.8 cpm (mean ± SD, hereinafter the same applies) in the hrHGF(-) samples, and 20916.9 ± 1574.2 cpm in the hrHGF(+) samples. Addition of hrHGF at a 2,000-fold mole concentration allowed to reduce binding of [¹²⁵I]HGF(11-83)-NH₂ (73 aa) to the cells by 20%. The reduction level was found to be statistically significant (P=0.040) (Fig. 8), indicating that [¹²⁵I]HGF(11-83)-NH₂ (73 aa) was bound to the HGF receptor of HuCCT1 cells.

### Example 6

### Determination of the HGF-receptor-expression site in myocardial ischemia model rats using [¹²⁵I]HGF(11-83)-NH₂ (73 aa)

### <Production of myocardial ischemia model>

CD(SD) IGS rats (10-week-old) were anesthetized by intraperitoneally administering pentobarbital Na (35 mg/kg). After tracheostomy, an artificial respirator was attached to the rat. The left chest was opened, and an electrocardiogram was measured before coronary ligation. The coronary artery and its peripheral tissue (surrounding several millimeters) were ligated with suture, and ischemic state was determined on the basis of ST rise and recorded by an electrocardiogram. After recording, the chest was closed, and the trachea and the skin were sutured.

### <Autoradiography>

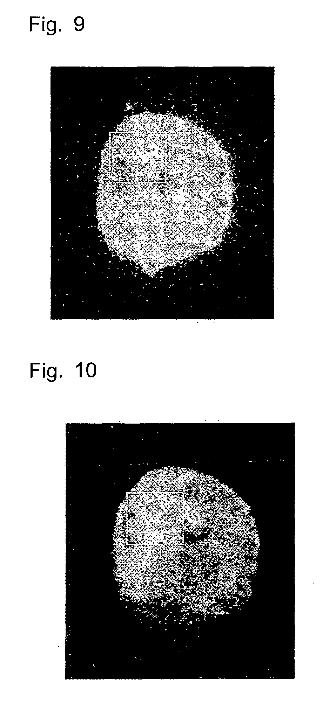

Three days after production of the myocardial ischemia model, a blood flow imaging agent ^{99m}Tc-MIBI (Cardiolight Injection; DAIICHI RADIOISOTOPE LABS., LTD.) and [¹²⁵I]HGF(11-83)-NH₂ (73 aa) were administered to each model rat. Blood flow was assessed through dual nuclide autoradiography and HE staining. ^{99m}Tc-MIBI was administered at 60 MBq through a femoral vein. Fifteen minutes after administration of ^{99m}Tc-MIBI, [¹²⁵I]HGF(11-83)-NH₂ (73 aa) was administered at 740 kBq through the femoral vein, and the heart was isolated after 15 minutes. On the day of the experiment, the sample was affixed to an imaging plate for 30 minutes, and a ^{99m}Tc-MIBI accumulation image was recorded by means of a bio-imaging analyzer BAS-1800 (Fig. 9). After 7-days attenuation period for ^{99m}Tc for seven days, the sample was set again to the imaging plate for seven days, and a [¹²⁵I]HGF(11-83)-NH₂ (73 aa) accumulation image was recorded by means of a bio-imaging analyzer BAS-1800 (Fig. 10).

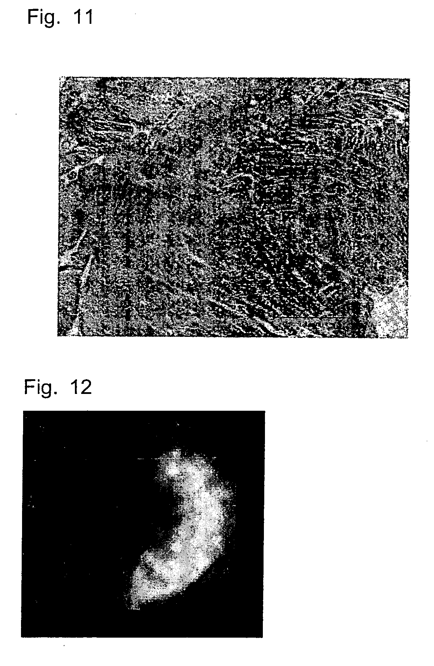

As a result of the dual nuclide autoradiography, accumulation of [¹²⁵I]HGF(11-83)-NH₂ (73 aa) (1.4 to 2.3 times accumulation density with respect to normal cardiac muscle) was observed around the infarct border zone at which reduced accumulation of ^{99m}TC-MIBI was observed. In conjunction with the result from HE staining of the serial sections, the [¹²⁵I]HGF(11-83)-NH₂ (73 aa) accumulation site was determined to correspond to the infarct border zone which is considered as an area excessively expressing HGF receptors (Fig. 11).

### Example 7

### Imaging of the HGF-receptor-expression site in a myocardial ischemia model using [¹²³I]HGF(11-83)-NH₂ (73 aa)

### <Production of myocardial ischemia model>

Wistar rats (8-week-old) were anesthetized with isoflurane (Forane (R), Abbott). After tracheostomy, an artificial respirator connected to an anesthetizer was attached to each rat. The left chest was opened, and an electrogardiogram was measured before coronary ligation. The coronary artery and its peripheral tissue were ligated with suture. During ligation, nylon filament was placed as a spacer between the coronary artery and the suture in order to weaken the ligation strength so as not to completely intercept the blood flow. Ischemic state was determined on the basis of ST rise and recorded by an electrocardiogram. After recording, the left chest was closed, and the trachea and the skin were sutured.

### <SPECT recording>

Under inhalation anesthesia with isoflurane, ^{99m}TC-MIBI (90 MBq) was injected into the left femoral vein of each myocardial ischemia model rat . Subsequently, [¹²³I]HGF(11-83)-NH₂ (73 aa) (100 MBq) prepared in a manner similar to that of Example 2 was administered through the right femoral vein. Thirty minutes after the administration, pinhole-SPECT imaging was conducted using a gamma camera (PRISM 2000, Shimadzu Corporation) (Imaging conditions: pinhole collimator, matrix sizes: 256 x 256, Mag 1.0, energy windows ^{99m}Tc: 140 keV 10% and ¹²³I: 159 keV 10%, 3° 60 sec/step). After imaging, the heart was isolated from the rat and pinhole-SPECT imaging was conducted ex vivo under the same imaging conditions.

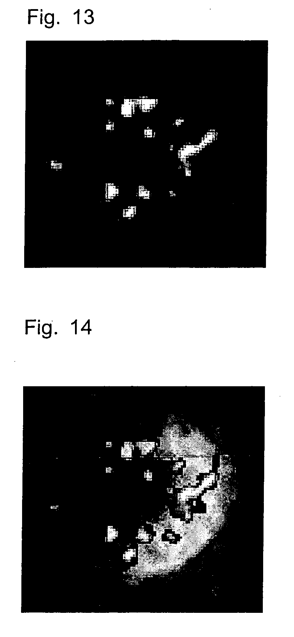

As a result, reduced blood flow was observed in a ^{99m}Tc-MIBI pinhole-SPECT images, indicating that a myocardial ischemia model was established (Fig. 12). In [¹²³I]HGF(11-83)-NH₂ (73 aa) pinhole-SPECT images, accumulation of [¹²³I]HGF(11-83)-NH₂ (73 aa) was observed at high level around the infarct border zone at which reduced accumulation of ^{99m}Tc-MIBI was observed (Fig. 13) . The pinhole-SPECT images also indicated that [¹²³I]HGF(11-83)-NH₂ (73 aa) was accumulated at high level in an area showing reduced blood-flow in which HGF receptors are considered to be expressed (Fig. 14).

## Claims

1. A diagnostic drug for a disease associated with a hepatocyte growth factor receptor, wherein the drug includes: a labeled product of a partial polypeptide of hepatocyte growth factor containing a heparin-binding domain; a labeled product of a polypeptide which consisting of an amino acid sequence in which one or several amino acid residues are substituted, deleted or added to the amino acid sequence of the partial polypeptide and having a heparin-binding activity.

2. The diagnostic drug as described in claim 1, wherein the heparin-binding domain is Cys39 to Cys65.

3. The diagnostic drug as described in claim 1 or 2, wherein the partial polypeptide contains Cys39 to Cys65 and has 4 to 40 amino acid residues at the N-terminal side and 4 to 110 amino acid residues at the C-terminal side of the Cys39 to Cys65, and wherein the labeled product thereof is labeled with a radioactive nuclide at an amino acid residue other than the Cys39 to Cys65.

4. The diagnostic drug as described in claim 1 or 2, wherein the partial polypeptide contains Cys39 to Cys65 and has 4 to 40 amino acid residues at the N- terminal side and/or the C- terminal side of the Cys39 to Cys65, and wherein the labeled product thereof is labeled with a radioactive nuclide at an amino acid residue other than the Cys39 to Cys65.

5. The diagnostic drug as described in claim 1 or 2, wherein the partial polypeptide contains Cys39 to Cys65 and has 4 to 30 amino acid residues at the N-terminal side and/or the C-terminal side of the Cys39 to Cys65, and wherein the labeled product thereof is labeled with a radioactive nuclide at an amino acid residue other than the Cys39 to Cys65.

6. The diagnostic drug as described in claim 1 or 2, wherein the partial polypeptide is a polypeptide consisting of the amino acid sequence represented by SEQ ID NO: 2, or a polypeptide consisting of an amino acid sequence in which one or several amino acid residues are substituted, deleted or added to said amino acid sequence and having a heparin-binding activity.

7. The diagnostic drug as described in any one of claims 1 to 6, which is a hepatocyte growth factor receptor imaging agent.

8. The diagnostic drug as described in any one of claims 1 to 7, wherein the disease associated with a hepatocyte growth factor receptor is an ischemic disease or a malignant tumor.

9. A therapeutic drug for a disease associated with a hepatocyte growth factor receptor, wherein the drug includes:
a partial polypeptide of hepatocyte growth factor containing a heparin-binding domain; a polypeptide which consisting of an amino acid sequence in which one or several amino acid residues are substituted, deleted or added to the amino acid sequence of the partial polypeptide and having a heparin-binding activity; a labeled product thereof; or a toxin-bound product thereof.

10. The therapeutic drug as described in claim 9, wherein the heparin-binding domain is Cys39 to Cys65.

11. The therapeutic drug as described in claim 9 or 10, wherein the partial polypeptide contains Cys39 to Cys65 and has 4 to 40 amino acid residues at the N-terminal side and 4 to 110 amino acid residues at the C-terminal side of the Cys39 to Cys65.

12. The therapeutic drug as described in claim 9 or 10, wherein the partial polypeptide contains Cys39 to Cys65 and has 4 to 40 amino acid residues at the N-terminal side and/or the C-terminal side of the Cys39 to Cys65.

13. The therapeutic drug as described in claim 9 or 10, wherein the partial polypeptide contains Cys39 to Cys65 and has 4 to 30 amino acid residues at the N-terminal side and/or the C-terminal side of the Cys39 to Cys65.

14. The therapeutic drug as described in claim 9 or 10, wherein the partial polypeptide is a polypeptide consisting of the amino acid sequence represented by SEQ ID NO: 2, or a polypeptide consisting of an amino acid sequence in which one or several amino acid residues are substituted, deleted or added to said amino acid sequence and having a heparin-binding activity.

15. The therapeutic drug as described in any one of claims 9 to 14, wherein the disease associated with a hepatocyte growth factor receptor is an ischemic disease or a malignant tumor.

16. Use of a labeled product of a partial polypeptide of hepatocyte growth factor containing a heparin-binding domain; or a labeled product of a polypeptide which consisting of an amino acid sequence in which one or several amino acid residues are substituted, deleted or added to the amino acid sequence of the partial polypeptide and having a heparin-binding activity, for producing a diagnostic drug for a disease associated with a hepatocyte growth factor receptor.

17. The use as described in claim 16, wherein the heparin-binding domain is Cys39 to Cys65.

18. The use as described in claim 16 or 17, wherein the partial polypeptide contains Cys39 to Cys65 and has 4 to 40 amino acid residues at the N-terminal side and 4 to 110 amino acid residues at the C-terminal side of the Cys39 to Cys65, and wherein the labeled product is labeled with a radioactive nuclide at an amino acid residue other than the Cys39 to Cys65.

19. The use as described in claim 16 or 17, wherein the partial polypeptide contains Cys39 to Cys65 and has 4 to 40 amino acid residues at the N-terminal side and/or the C-terminal side of the Cys39 to Cys65, and wherein the labeled product thereof is labeled with a radioactive nuclide at an amino acid residue other than the Cys39 to Cys65.

20. The use as described in claim 16 or 17, wherein the partial polypeptide contains Cys39 to Cys65 and has 4 to 30 amino acid residues at the N-terminal side and/or the C-terminal side of the Cys39 to Cys65, and wherein the labeled product thereof is labeled with a radioactive nuclide at an amino acid residue other than the Cys39 to Cys65.

21. The use as described in claim 16 or 17, wherein the partial polypeptide is a polypeptide consisting of the amino acid sequence represented by SEQ ID NO: 2, or a polypeptide consisting of an amino acid sequence in which one or several amino acid residues are substituted, deleted or added to said amino acid sequence and having a heparin-binding activity.

22. The use as described in any one of claims 16 to 21, wherein the diagnostic drug is a hepatocyte growth factor receptor imaging agent.

23. The use as described in any one of claims 16 to 21, wherein the disease associated with a hepatocyte growth factor receptor is an ischemic disease or a malignant tumor.

24. Use of a partial polypeptide of hepatocyte growth factor containing a heparin-binding domain; a polypeptide which consisting of an amino acid sequence in which one or several amino acid residues are substituted, deleted or added to the amino acid sequence of the partial polypeptide and having a heparin-binding activity; a labeled product thereof; or a toxin-bound product thereof, for producing a therapeutic drug for a disease associated with a hepatocyte growth factor receptor.

25. The use as described in claim 24, wherein the heparin-binding domain is Cys39 to Cys65.

26. The use as described in claim 24 or 25, wherein the partial polypeptide contains Cys39 to Cys65 and has 4 to 40 amino acid residues at the N-terminal side and 4 to 110 amino acid residues at the C-terminal side of the Cys39 to Cys65.

27. The use as described in claim 24 or 25, wherein the partial polypeptide contains Cys39 to Cys65 and has 4 to 40 amino acid residues at the N- terminal side and/or the C-terminal side of the Cys39 to Cys65.

28. The use as described in claim 24 or 25, wherein the partial polypeptide contains Cys39 to Cys65 and has 4 to 30 amino acid residues at the N-terminal side and/or the C-terminal side of the Cys39 to Cys65.

29. The use as described in claim 24 or 25, wherein the partial polypeptide is a polypeptide consisting of the amino acid sequence represented by SEQ ID NO: 2, or a polypeptide consisting of an amino acid sequence in which one or several amino acid residues are substituted, deleted or added to said amino acid sequence and having a heparin-binding activity.

30. The use as described in any one of claims 24 to 29, wherein the disease associated with a hepatocyte growth factor receptor is an ischemic disease or a malignant tumor.

31. A method for diagnosing a disease associated with a hepatocyte growth factor receptor, wherein the method comprises administering a labeled product to a subject in need thereof; and detecting the labeled product, wherein the labeled product is a labeled product of a partial polypeptide of hepatocyte growth factor containing a heparin-binding domain or a labeled product of a polypeptide which consisting of an amino acid sequence in which one or several amino acid residues are substituted, deleted or added to the amino acid sequence of the partial polypeptide and having a heparin-binding activity.

32. The diagnosis method as described in claim 31, wherein the heparin-binding domain is Cys39 to Cys65.

33. The diagnosis method as described in claim 31 or 32, wherein the partial polypeptide contains Cys39 to Cys65 and has 4 to 40 amino acid residues at the N-terminal side and 4 to 110 amino acid residues at the C-terminal side of the Cys39 to Cys65, and wherein the labeled product is labeled with a radioactive nuclide at an amino acid residue other than the Cys39 to Cys65.

34. The diagnosis method as described in claim 31 or 32, wherein the partial polypeptide contains Cys39 to Cys65 and has 4 to 40 amino acid residues at the N-terminal side and/or the C-terminal side of the Cys39 to Cys65, and wherein the labeled product thereof is labeled with a radioactive nuclide at an amino acid residue other than the Cys39 to Cys65.

35. The diagnosis method as described in claim 31 or 32, wherein the partial polypeptide contains Cys39 to Cys65 and has 4 to 30 amino acid residues at the N-terminal side and/or the C-terminal side of the Cys39 to Cys65, and wherein the labeled product thereof is labeled with a radioactive nuclide at an amino acid residue other than the Cys39 to Cys65.

36. The diagnosis method as described in claim 31 or 32, wherein the partial polypeptide is a polypeptide consisting of the amino acid sequence represented by SEQ ID NO: 2, or a polypeptide consisting of an amino acid sequence in which one or several amino acid residues are substituted, deleted or added to said amino acid sequence and having a heparin-binding activity.

37. The diagnosis method as described in any one of claims 31 to 36, which is a hepatocyte growth factor receptor imaging method.

38. The diagnosis method as described in any one of claims 31 to 37, wherein the disease associated with a hepatocyte growth factor receptor is an ischemic disease or a malignant tumor.

39. A method for treatment of a disease associated with a hepatocyte growth factor receptor, wherein the method comprises administering a partial polypeptide of hepatocyte growth factor containing a heparin-binding domain; a polypeptide which consisting of an amino acid sequence in which one or several amino acid residues are substituted, deleted or added to the amino acid sequence of the partial polypeptide and having a heparin-binding activity; a labeled thereof; or a toxin-bound product thereof, to a subject in need thereof.

40. The treatment method as described in claim 39, wherein the heparin-binding domain is Cys39 to Cys65.

41. The treatment method as described in claim 39 or 40, wherein the partial polypeptide contains Cys39 to Cys65 and has 4 to 40 amino acid residues at the N-terminal side and 4 to 110 amino acid residues at the C-terminal side of the Cys39 to Cys65.

42. The treatment method as described in claim 39 or 40, wherein the partial polypeptide contains Cys39 to Cys65 and has 4 to 40 amino acid residues at the N-terminal side and/or the C-terminal side of the Cys39 to Cys65.

43. The treatment method as described in claim 39 or 40, wherein the partial polypeptide contains Cys39 to Cys65 and has 4 to 30 amino acid residues at the N-terminal side and/or the C-terminal side of the Cys39 to Cys65.

44. The treatment method as described in claim 39 or 40, wherein the partial polypeptide is a polypeptide consisting of the amino acid sequence represented by SEQ ID NO: 2, or a polypeptide consisting of an amino acid sequence in which one or several amino acid residues are substituted, deleted or added to said amino acid sequence and having a heparin-binding activity.

45. The treatment method as described in any one of claims 39 to 44, wherein the disease associated with a hepatocyte growth factor receptor is an ischemic disease or a malignant tumor.

46. A method for screening a preventive or therapeutic drug for a disease associated with a hepatocyte growth factor receptor, wherein the method comprises selecting a substance which inhibits binding of a hepatocyte growth factor receptor to a partial polypeptide of hepatocyte growth factor containing a heparin-binding domain; to a polypeptide which consisting of an amino acid sequence in which one or several amino acid residues are substituted, deleted or added to the amino acid sequence of the partial polypeptide and having a heparin-binding activity; or to a labeled product thereof.

47. The screening method as described in claim 46, wherein the heparin-binding domain is Cys39 to Cys65.

48. The screening method as described in claim 46 or 47, wherein the partial polypeptide contains Cys39 to Cys65 and has 4 to 40 amino acid residues at the N-terminal side and 4 to 110 amino acid residues at the C-terminal side of the Cys39 to Cys65.

49. The screening method as described in claim 46 or 47, wherein the partial polypeptide contains Cys39 to Cys65 and has 4 to 40 amino acid residues at the N-terminal side and/or the C-terminal side of the Cys39 to Cys65.

50. The screening method as described in claim 46 or 47, wherein the partial polypeptide contains Cys39 to Cys65 and has 4 to 30 amino acid residues at the N-terminal side and/or the C- terminal side of the Cys39 to Cys65.

51. The screening method as described in claim 46 or 47, wherein the partial polypeptide is a polypeptide consisting of the amino acid sequence represented by SEQ ID NO: 2, or a polypeptide consisting of an amino acid sequence in which one or several amino acid residues are substituted, deleted or added to said amino acid sequence and having a heparin-binding activity.

52. The screening method as described in claim 46 or 47, wherein the disease associated with a hepatocyte growth factor receptor is an ischemic disease or a malignant tumor.

53. A preventive or therapeutic drug for a disease associated with a hepatocyte growth factor receptor, which is screened by the method as described in any one of claims 46 to 52.
